# EUROPEAN PATENT SPECIFICATION

(11) **EP 3 363 472 B1**
(45) Date of publication and mention of the grant of the patent: **05.08.2020**
(21) Application number: 16855488.9
(22) Date of filing: 13.10.2016
(51) Int. Cl.: A61L 2/14, B65B 55/10, H05H 1/24

(54) **STERILIZATION METHOD WITH REACTIVE OXYGEN GENERATED BY PLASMA**
STERILISATIONSVERFAHREN MITTELS PLASMASERZEUGTEN REAKTIVEN SAUERSTOFFS
PROCÉDÉ DE STÉRILISATION UTILISANT OXYGÈNE RÉACTIF GÉNÉRÉ PAR PLASMA

(30) Priority: 13.10.2015 JP 2015202316
(43) Date of publication of application: 22.08.2018
(73) Proprietor: Suntory Holdings Limited, Osaka-shi, Osaka 530-8203 (JP)
(72) Inventor: HIGASHIYAMA, Kenichi, Kyoto 619-0284 (JP); TOMINAGA, Kenta, Kyoto 619-0284 (JP); HIRAYAMA, Yuji, Kyoto 619-0284 (JP); YOSHIHARA, Kazuki, Kyoto 619-0284 (JP)
(74) Representative: Mewburn Ellis LLP
(86) International application number: PCT/JP2016/080431
(87) International publication number: WO 2017/065235

(56) References cited:
- JP-A- H09 122 211
- JP-A- H11 342 917
- JP-A- 2000 326 935
- JP-A- 2000 326 935
- JP-A- 2006 331 763
- JP-A- 2007 145 407
- JP-A- 2007 145 407
- JP-A- 2007 268 252
- US-A1- 2013 202 496
- US-A1- 2013 319 460

## Description

### TECHNICAL FIELD

The present invention relates to a sterilization method. More particularly, the present invention relates to a sterilization method including carrying out sterilization treatment including irradiating reactive oxygen.

### BACKGROUND ART

Containers for foods or beverages (foodstuff) or the like are required to be sterilized on internal and external sides thereof. As a conventional sterilization method, a method using an aqueous hydrogen peroxide or a chemical has been known. However, there are some disadvantages that the aqueous hydrogen peroxide or chemical is likely to remain, so that the development of substitute techniques has been studied.

For example, Patent Publication 1 discloses a method including generating a plasma jet using discharge in a fluid, contacting a surface of an object with the plasma jet, and carrying out sterilization (disinfection) by way of energy transfer from the plasma jet to the surfaces. The plasma jet used in this publication is generated by atmospheric electric discharge in a process gas containing oxygen, preferably the air.

Patent Publication 2 discloses a dishwasher with a plasma generation device.

Patent Publication 3 discloses a sterilisation method with the features defined in the preamble of claim 1.

### RELATED ART REFERENCES

### PATENT PUBLICATIONS

Patent Publication 1: Japanese Unexamined Patent Publication No. 2009-519799
Patent Publication 2: US Patent Publication No. US 2013/0319460 A1
Patent Publication 3: Japanese Unexamined Publication No. 2000-326935

### SUMMARY OF THE INVENTION

### PROBLEMS TO BE SOLVED BY THE INVENTION

In general, reactive oxygen species (ROS) such as superoxide radical (•O₂⁻), hydrogen peroxide (H₂O₂), or hydroxy radical (HO•) exhibit an excellent sterilization action due to their strong oxidizing actions, and these reactive oxygen species are produced mainly from oxygen molecules or water in the air. Specifically, for example, hydroxy radicals are obtained by a reaction of water molecules with plasma electrons.

However, the sustaining of the sterilization actions of reactive oxygen is unknown, and further techniques have been demanded.

An object of the present invention is to provide a sterilization method having excellent sterilization effects.

### MEANS TO SOLVE THE PROBLEMS

The present invention defines a sterilisation method according to claim 1.

Preferred embodiments of the present invention are defined in the dependent claims.

### EFFECTS OF THE INVENTION

The sterilization method of the present invention exhibits some excellent effects that the sterilization effects are excellent. Also, a chemical or the like which has been used in conventional sterilization does not remain because the sterilization is carried out with a fluid, which leads to simplifications of processing steps, whereby productivity can be remarkably improved.

### BRIEF DESCRIPTION OF THE DRAWINGS

[FIG. 1] FIG. 1 is a schematic view showing one embodiment of a sterilization apparatus used for the method of the present invention.
[FIG. 2] FIG. 2 is a view showing a method of irradiating a reactive oxygen which was carried out in Test Example, wherein the left view is Example 1, and the right view is Comparative Example 1.
[FIG. 3] FIG. 3 is a view showing a sterilization apparatus used in Test Example (Example 2).
[FIG. 4] FIG. 4 is a view showing one example, useful for the understanding of the present invention, of moving an object to be sterilized in and out of a container (chamber) in the sterilization apparatus used in Test Example (Example 2).

### MODES FOR CARRYING OUT THE INVENTION

The sterilization method of the present disclosure is, for example, a method in which reactive oxygen obtained by generating plasma using an alternating current, and generating a reactive oxygen from the plasma obtained is used, characterized in that the sterilization method includes carrying out sterilization including allowing an object to be sterilized to stand within a container which is previously irradiated with reactive oxygen. The reactive oxygen used in the present invention is generated by plasma, preferably by a reaction of plasma and steam, and it is assumed that a radical formation reaction continues by detaining these reactive oxygens within a closed space, whereby the sterilization effects are sustained. However, these assumptions do not limit the present invention thereto. Here, in the present invention, the term "sterile" or "sterilization" means breaking of live bodies of microbes or removal thereof from surfaces of objects to be sterilized, which, for example, includes disinfection, sterilization or sterile filtration.

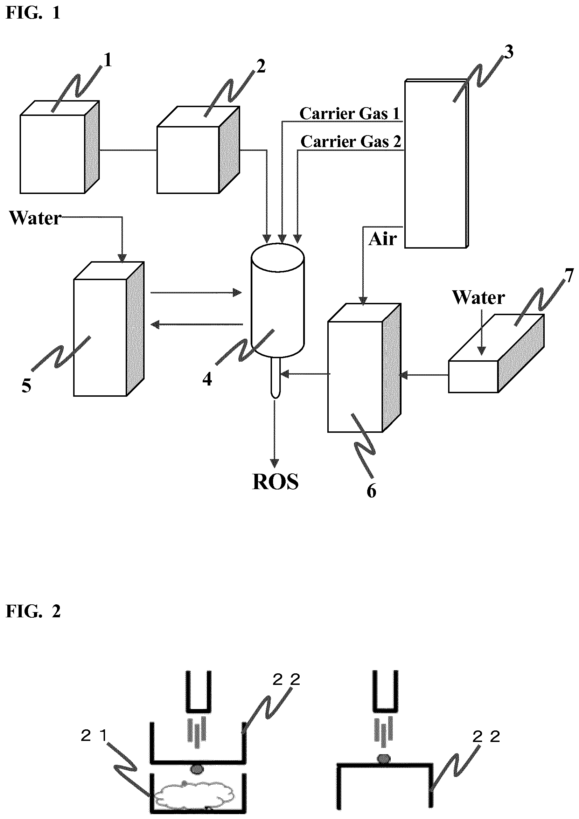

For performing the method of the present invention, it is preferable to use sterilization apparatus comprising the following constitution, and one embodiment thereof will be explained more specifically based on FIG. 1. Here, the sterilization apparatus shown in FIG. 1 is merely one embodiment of the present disclosure, without intending to limit the present disclosure thereto.

As shown in FIG. 1, the sterilization apparatus usable for the present invention comprises each of the units of an inlet unit 1 for alternating current, a high-voltage unit 2, an inlet unit 3 for gas flow, a nozzle 4, a chilling unit 5 of the nozzle, an inlet unit 6 for steam flow to the nozzle, and an inlet unit 7 for water flow to the inlet unit for steam flow.

The inlet unit 1 for alternating current is a source of generating electric charges of plasma discharge. The alternating current to be supplied is not particularly limited, and the alternating current includes, for example, ones generated at a frequency of from 10 to 15 kHz, and a voltage of from 200 to 500 V or so, which can be properly set in accordance with known techniques. The level of amperes of the alternating current is not particularly limited, and the level can be properly adjusted depending upon the specifications of the inlet device; for example, an alternating current of 11 A may be used. In the present invention, direct current can be used in place of alternating current but alternating current is preferred from the viewpoint of adjusting voltage.

The high-voltage unit 2 is a device which is connected with the inlet unit 1 for alternating current, and increases voltage of the alternating current supplied from the unit 1, and any devices that are capable of increasing voltages can be used without particular problems. In addition, the high-voltage unit may be integrated with the unit 1. The increased voltage is not particularly limited, and is, for example, from 10 to 30 kV or so.

The inlet unit 3 for gas flow is a device for inlet of gas flows of various gases to each of a nozzle 4 and an inlet unit 6 for steam flow, and a known inlet device for gas flow can be used.

Specifically, a carrier gas for generating plasma is allowed to flow to a nozzle 4. As the carrier gas, the air, oxygen, nitrogen, argon, helium, and mixtures thereof can be used, among which it is preferable to use two kinds of the air and oxygen. The flow rate of the carrier gas is not unconditionally set, depending upon the size, shape, or the like of the nozzle 4. For example, an embodiment includes allowing the air to flow at a rate of 6 L/min, and oxygen to flow at a rate of 3 L/min.

The air for mixing with the steam needed when producing reactive oxygen from plasma is allowed to flow to the inlet unit 6 for steam flow. By using a water-containing gas in which the steam is mixed with the air, the mixing of the plasma and steam is accelerated, whereby hydroxy radicals can be efficiently produced from the steam. The flow rate of the air to the inlet unit 6 for steam flow is the same as the flow rate of the water-containing gas to the nozzle 4. For example, an embodiment of allowing the air to flow at a rate of 3 L/min is exemplified. Here, the air as used herein refers to a gas of which relative humidity is from 0 to 10% by volume or so at 20°C.

The nozzle 4 is a device of irradiating reactive oxygen obtained by generating plasma, which is also referred to as a reactive oxygen irradiation unit. The device comprises an internal electrode and an external electrode, and an elevated voltage is applied between both the electrodes from the high-voltage unit 2, whereby making it possible to generate an electric field. In addition, the internal electrode may be connected with a coil, so that an even larger electric field can be formed. The shape, size or the like of the coil can be adjusted in accordance with the technical common knowledge of one of ordinary skill in the art.

In addition, the device comprises a gas inlet port and a reactive oxygen irradiation port, wherein the gas inlet port exists at an end of an opposite side to an end part at which the reactive oxygen irradiation inlet exists. Moreover, the gas inlet port is connected with a pipe from the inlet unit 3 for gas flow, wherein plasma is produced by passing a carrier gas through the electric field generated as mentioned above. Since the plasma produced as described above is also a fluid, the plasma may also be referred to as a plasma jet. On the other hand, the reactive oxygen irradiation port has a tubular structure or a conical structure that is tapered toward a discharge opening, and connected with a pipe for allowing a water-containing gas to flow from the inlet unit 6 for steam flow at any of the parts before reaching the discharge opening, at which a reactive oxygen would be produced by a reaction with the plasma produced above, and irradiated from the discharge opening of the reactive oxygen irradiation port.

The nozzle 4 is not particularly limited in the shape or size so long as the nozzle has the above parts. For example, a structure comprising a gas inlet port arranged at an upper end of a cylindrical structure, and a reactive oxygen irradiation port having a tubular structure having a diameter smaller than the diameter of the apparatus at a lower end thereof is exemplified. The cylindrical structure may form a layered structure, and, for example, a structure in which a coil is formed in the surroundings of the tube through which a carrier gas passes, and optionally a layer of an insulation material is further formed in the surroundings of the coil is exemplified. The tube is not particularly limited so long as the tube is an electroconductive material, and known materials in the art can be used. In addition, the insulation material is not particularly limited, and a known insulation material in the art can be used.

The chilling unit 5 of the nozzle is a device for allowing a chilling water to flow to the nozzle 4, and a known device for chilling water flow can be used. Since the nozzle 4 generates heat by applying a high voltage, it is preferable to chill the nozzle. As the chilling water, waters at temperatures of, for example, 5°C or so are preferably used, and the chilling water may be circulated between the nozzle 4 and the chilling unit 5. The flow rate of the chilling water can be properly adjusted so that the surface temperature of the nozzle 4 is controlled to 25°C or lower. Here, the surface temperature of the nozzle 4 can be measured with a contact-type thermometer.

The inlet unit 6 for steam flow to the nozzle is a device of allowing a water-containing gas to flow to the nozzle 4, and the inlet unit is connected to a reactive oxygen irradiation port of the nozzle 4 as mentioned above. When the water-containing gas is allowed to flow, first, water from an inlet unit 7 for water flow is heated with electric heating wires installed therein to produce steam, and a mixture of the steam with the air allowed to flow from the inlet unit 3 for gas flow is allowed to flow to the nozzle 4 as a water-containing gas. Here, the inlet unit 7 for water flow may be integrated with the inlet unit 6 for steam flow. The heating temperature of the electric heating wires can be properly adjusted depending upon the flow rate of water, which is exemplified by, for example, 300°C. Also, the flow rate of water from the inlet unit 7 for water flow can be adjusted depending upon the amount of steam needed to produce reactive oxygen. In the present invention, the flow rate of water is preferably 0.5 mL/min or more, and more preferably 1.0 mL/min or more, from the viewpoint of containing a water content in the reactive oxygen-containing gas in an amount equal to or greater than the saturated steam. In addition, although the upper limit is not particularly set, the flow rate of water is preferably 6 mL/min or less, and more preferably 5 mL/min or less. The steam thus obtained is mixed with the air allowed to flow from the inlet unit 3 for gas flow in a volume ratio (steam/the air) of from 0.2 to 2.5 or so, and the water-containing air is allowed to flow to a reactive oxygen irradiation port of the nozzle 4. The mixing volume ratio of steam to the air can be modified by fluctuating the flow rate of water mentioned above, and the amount of steam contained in the water-containing air can be increased by increasing the flow rate of water. Examples of the mixing volume ratio of the plasma jet produced in the nozzle 4 to the water-containing gas allowed to flow from the inlet unit 6 for steam flow [plasma jet/water-containing gas] include from 0.8 to 2.6.

The reactive oxygen is irradiated as described above, and the present disclosure is characterized in that the reactive oxygen is irradiated within a container 8 in which an object to be sterilized is previously placed, specifically a sterilization chamber, not directly onto an object to be sterilized. In other words, the sterilization method of the present disclosure includes irradiating reactive oxygen within a container such as a chamber, not directly irradiating to an object to be sterilized, for preferably at least within 10 seconds. Depending upon the kind of container, the sterilization is carried out by placing an object to be sterilized under an atmosphere filled with a reactive oxygen within 2 minutes or so. After the object to be sterilized is loaded into a container, the interior of the container may be made into a closed space, and a closed space may be formed by placing a cover on the container while placing the object.

The placing time is preferably 10 seconds or longer, and more preferably 30 seconds or longer. Although the upper limit is not particularly set, the placing time is, for example, 60 minutes or less, preferably 30 minutes or less, and more preferably 10 minutes or less, from the viewpoint of operability. Also, the temperature within the container while placing the object is not particularly limited, and the temperature is, for example, from 2° to 40°C.

The container is not particularly limited so long as an object to be sterilized can be contained, and preferred is a container which can have a cover over it.

Here, the sterilization apparatus usable for the present invention may further comprise other units besides the units mentioned above. Examples of other units include an irradiation platform on which an object to be sterilized is placed, a shielding wall for preventing the diffusion of reactive oxygen, and the like. The irradiation platform on which an object to be sterilized is placed is not particularly limited, so long as an object to be sterilized can be placed. It is preferable that the object can be placed at a temperature of equal to or lower than an ordinary temperature (40°C), from the viewpoint of allowing hydroxy radicals not to decompose due to high temperatures.

Thus, hydroxy radicals are continuously produced under an atmosphere filled with reactive oxygen, which in turn makes it possible to sustain excellent sterilization activity. Also, since reactive oxygen is a fluid, even a three-dimensional structured object can be sterilized, thereby exhibiting some excellent effects in that residues do not remain on edges or corners.

The reactive oxygen to be irradiated is warm due to electric discharge within the nozzle 4 or the water-contained gas from the inlet unit 6 for steam flow, the temperature of which is from about 50° to about 80°C. Because of this warmth, the heated load of the irradiated object is considered to be small. Here, the temperature of the reactive oxygen refers to a temperature of reactive oxygen at a discharge opening of the reactive oxygen irradiation port that is measured with a thermocouple thermometer.

In addition, a temperature difference between the reactive oxygen and the surface of the object to be sterilized is, for example, 10°C or more, and more preferably from 25° to 40°C, from the viewpoint of increasing a reactivity of radicals. The temperature of the surface of the object to be sterilized as used herein refers to a temperature of an object to be sterilized that is measured with a contact-type thermometer.

The irradiation speed can be adjusted according to the flow rate of the gas and the shape of the reactive oxygen irradiation port, and, for example, the irradiation speed includes 50,000 mm/sec. The irradiation time may be adjusted in accordance with a volume of a container and an opening ratio, so that the gas is sufficiently filled within the container, and an irradiation time is exemplified by, for example, from 0.05 to 1 second, and the irradiation time may be 2 minutes or so, depending upon a container.

In addition, it is preferable that the distance between the reactive oxygen irradiation port and the surface of the object to be sterilized is, for example, from 5 to 50 mm.

The sterilization method of the present invention includes an embodiment comprising irradiating a reactive oxygen to an object having a recessed portion, for example, a bottle cap or the like, within the recessed portion, stacking an object to be sterilized on top thereof to place a cover over the above recessed portion, and carrying out sterilization within the closed space formed, characterised in that the reactive oxygen is obtained by generating plasma.

Specifically, a sterilization method of a bottle cap for sealing an open portion of a container includes a sterilization method characterized by placing a top side of the bottle cap which is an object to be sterilized facing downwards, irradiating a reactive oxygen within a dent portion opening towards an upward direction, thereafter stacking a fresh bottle cap on top thereof to place a cover in the dent portion, and carrying out sterilization within a closed space formed. Here, a bottle cap refers to, for example, a bottle cap. More specifically, for example, as shown in the left view of FIG. 2, reactive oxygen is irradiated to a recessed portion of a bottle cap (bottle cap 21), i.e. an internal side of the bottle cap 21, and a fresh bottle cap (bottle cap 22) is stacked on to place a cover, so that both the internal side of the bottle cap 21 and the top side of the bottle cap 22 can be sterilized. Here, the specifications and the methods of use and the like of the sterilization apparatus are as described in the section of the sterilization method mentioned above. The placing time of the bottle cap or the like can be properly set referring to the previous section. In this case, the bottle caps can be stacked in plurality vertically, in the same direction (i.e. recessed portions are the lower sides in all cases).

### EXAMPLES

The present invention will be described more specifically by means of Examples given hereinbelow, without intending to limit the present invention thereto.

### TEST EXAMPLE 1

The influences of the manners of irradiation of reactive oxygen depending upon the sterilization apparatuses usable in the present invention were studied.

### Preparation of Bacterial Solution and Preparation of Bacteria-Inoculated Bottle Cap

Using a bacterial solution of fibroblast bacterium *Bacillus atrophaeus*, bacterial solutions of various concentrations (3 standards within the concentration range of from 2 × 10³ to 2 × 10⁸ CFU/mL) were prepared. The obtained bacterial solution was inoculated in a top side (external side of bottom) of a resin bottle cap (material: polyethylene) as shown in FIG. 2 in an amount of 1 µL × 1 spot for each bottle cap (each concentration n = 5). Here, the inoculated resin bottle cap which was allowed to stand in a sterile petri dish for 24 hours to dryness was used.

### Irradiation 1 of Reactive Oxygen (Example 1: Indirect Irradiation)

Using the sterilization apparatus shown in FIG. 1, a resin bottle cap other than the inoculated resin bottle cap was irradiated with reactive oxygen onto the interior for 1 second per bottle cap, and immediately after the irradiation, the inoculated resin bottle cap was placed on top so that the outside of the bottom of the inoculated resin cap was covered with the recessed portion of the resin bottle cap irradiated with reactive oxygen, and the resin caps were allowed to stand for 30 minutes (25°C). The inoculated bottle cap after being allowed to stand was collected on a sterile petri dish. Here, the operating conditions of the sterilization apparatus were as follows.

### Operating Conditions of Sterilization Apparatus

Inlet unit 1 for alternating current: frequency: 14 kHz, voltage: 300 V, electric current: 11A
High-voltage unit 2: The raised voltage: 20 kV
Inlet unit 3 for gas flow: flow rate of air: 6 L/min, flow rate of oxygen: 3 L/min (hereinabove, go to nozzle 4), flow rate of air: 3 L/min (go to inlet unit 6 for steam flow)
Nozzle 4: temperature of reactive oxygen irradiation: 51°C, irradiation speed: 50,000 mm/sec
Chilling unit 5: chilling water: 5°C
Inlet unit 6 for steam flow: electric heating wires: 300°C, flow rate of water-containing gas: 4.5 L/min (flow rates of plasma jet/water-containing gas flow (volume ratio) = 9/4.5)
Inlet unit 7 for water flow: flow rate of water: 1.2 mL/min

### Irradiation 2 of Reactive Oxygen (Comparative Example 1: Direct Irradiation)

Using the sterilization apparatus shown in FIG. 1, an inoculated resin bottle cap was irradiated with reactive oxygen onto an external side of the bottom of the inoculated bottle cap at a distance 30 mm away from a top side for 1 second per bottle cap, and the irradiated bottle cap was collected on a sterile petri dish. The operating conditions of the sterilization apparatus were the same as detailed above.

### Measurement of Sterilization Activity Values

The collected resin bottle cap was taken out of the sterile petri dish, and 5 mL of TSA liquid medium (manufactured by BD Falcon) was injected to the bottle cap, and cultured at 35°C, a temperature suitable for proliferation of microbes for 3 days. After the cultivation, the number of bottle caps in which media became turbid due to microbial proliferation was counted as positive, and the sterilization activity value LRV (Log Reduction Value) was calculated according to the most probable number method (MPN method). The results are shown in Table 1. Here, the "D" value showing the sterilization activity is expressed by common logarithm (LOG value) of the number of bacteria per bottle cap, which is a value obtained by subtracting the number of bacteria after the treatment (LOG value) from the number of bacteria before the treatment (LOG value). It is shown that the larger the number, the higher the sterilization activity, and that if the value is 4.5 D or more, there are no problems as the sterilization treatment of food containers.

### Irradiation 3 of Reactive Oxygen (Example 2: Indirect Irradiation)

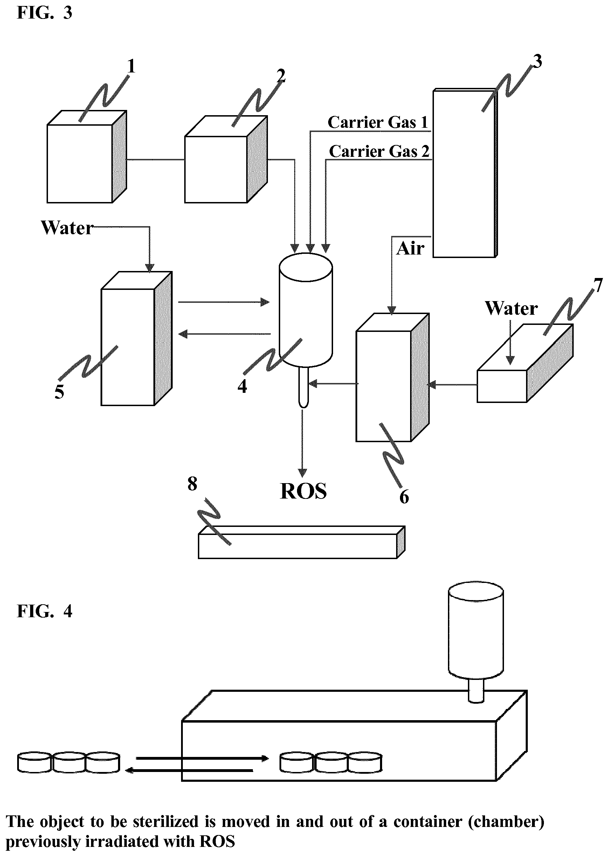

Using the sterilization apparatus shown in FIG. 3 (the details of the chamber are shown in FIG. 4), a gas was previously irradiated into a container (chamber) for 1 minute to sufficiently fill the container with the gas, and an inoculated resin bottle cap was then loaded into the container (chamber), and allowed to stand for 1 minute (25°C). The irradiated bottle cap was collected on a sterile petri dish. The operating conditions of the sterilization apparatus were as follows.

### Operating Conditions of Sterilization Apparatus

Inlet unit 1 for alternating current: frequency: 14 kHz, voltage: 300 V, electric current: 11A
High-voltage unit 2: The raised voltage: 20 kV
Inlet unit 3 for gas flow: flow rate of air: 6 L/min, flow rate of oxygen: 3 L/min (hereinabove, go to nozzle 4), flow rate of air: 3 L/min (go to inlet unit 6 for steam flow)
Nozzle 4: temperature of reactive oxygen irradiation: 51°C, irradiation speed: 50,000 mm/sec
Chilling unit 5: chilling water: 5°C
Inlet unit 6 for steam flow: electric heating wires: 300°C, flow rate of water-containing gas: 4.5 L/min (flow rates of plasma jet/water-containing gas flow (volume ratio) = 9/4.5)
Inlet unit 7 for water flow: flow rate of water: 0.2 mL/min
Container (chamber capacity) 8: 4 L

**Table 1**

| | Irradiation Method | LRV, D Value |
|---|---|---|
| | | |
| Comp. Ex. 1 | Direct Irradiation | 1.9 D or less |
| Ex. 1 | Indirect Irradiation | 4.9 D |
| Ex. 2 | Indirect Irradiation | 6.0 D |

It is suggested from the comparison of Example 1 with Comparative Example 1 that excellent sterilization effects are obtained even when not directly irradiated to the bacteria. Also, it can be seen from Example 2 that excellent sterilization effects are obtained even when an object to be sterilized is allowed to stand in a chamber to which irradiation is previously carried out.

The scope of the invention is defined by the appended claims.

### INDUSTRIAL APPLICABILITY

The sterilization method of the present invention shows excellent sterilization activity, so that it can be suitably used in, for example, sterilization of containers for foodstuff, bottle caps sealing the openings of the containers, medical devices, foodstuff such as vegetables and meat, and the like.

### EXPLANATION OF NUMERALS

- 1: inlet unit for alternating current
- 2: high-voltage unit
- 3: inlet unit for gas flow
- 4: nozzle
- 5: chilling unit
- 6: inlet unit for steam flow
- 7: inlet unit for water flow
- 8: chamber
- 21: bottle cap filled with reactive oxygen in the interior side
- 22: inoculated resin bottle cap

## Claims

1. A sterilization method comprising irradiating a first object to be sterilized comprising a recessed portion with a reactive oxygen at the recessed portion, stacking a second object to be sterilized on top thereof to cover the recessed portion so as to form a closed space and carrying out sterilization within this formed closed space;
**characterised in that** the reactive oxygen is obtained by generating plasma.

2. A sterilization method according to claim 1, wherein the reactive oxygen is obtained by generating plasma using an alternating current, and generating reactive oxygen from the plasma obtained.

3. A sterilization method according to claim 1 or 2, wherein the reactive oxygen is generated by a reaction of plasma and steam.

4. A sterilization method according to claim 3, wherein the steam is mixed with air in a ratio of 0.2 to 2.5 of steam:air.

5. A sterilization method according to any one of claims 1 to 4, wherein the plasma is a plasma jet.

6. A sterilization method according to any one of claims 3 to 5, wherein the reactive oxygen has a temperature of 50°C to 80°C.

7. A sterilization method according to any one of claims 1 to 6, the temperature difference between the reactive oxygen and the surface of the first object to be sterilised is 10°C or more.

8. A sterilization method according to any one of claims 1 to 7, wherein the first and second objects are bottle caps.

## Patentansprüche

1. Sterilisationsverfahren, umfassend das Bestrahlen eines ersten zu sterilisierenden Gegenstands, der einen vertieften Abschnitt umfasst, mit reaktivem Sauerstoff an dem vertieften Abschnitt, das Stapeln eines zweiten zu sterilisierenden Gegenstands auf diesen, um den vertieften Abschnitt so zu bedecken, um einen geschlossenen Raum auszubilden, und das Durchführen von Sterilisation innerhalb dieses gebildeten geschlossenen Raums;
**dadurch gekennzeichnet, dass** der reaktive Sauerstoff durch das Erzeugen von Plasma erhalten wird.

2. Sterilisationsverfahren nach Anspruch 1, wobei der reaktive Sauerstoff durch das Erzeugen von Plasma unter Verwendung von Wechselstrom erhalten wird, und das Erzeugen von reaktivem Sauerstoff aus dem erhaltenen Plasma.

3. Sterilisationsverfahren nach Anspruch 1 oder 2, wobei der reaktive Sauerstoff durch eine Reaktion von Plasma und Dampf erzeugt wird.

4. Sterilisationsverfahren nach Anspruch 3, wobei der Dampf in einem Verhältnis von 0,2 bis 2,5 von Dampf zu Luft mit Luft vermischt wird.

5. Sterilisationsverfahren nach einem der Ansprüche 1 bis 4, wobei das Plasma ein Plasmastrahl ist.

6. Sterilisationsverfahren nach einem der Ansprüche 3 bis 5, wobei der reaktive Sauerstoff eine Temperatur von 50 °C bis 80 °C aufweist.

7. Sterilisationsverfahren nach einem der Ansprüche 1 bis 6, wobei die Temperaturdifferenz zwischen dem reaktiven Sauerstoff und der Oberfläche des ersten zu sterilisierenden Gegenstands 10 °C oder mehr beträgt.

8. Sterilisationsverfahren nach einem der Ansprüche 1 bis 7, wobei der erste Gegenstand und der zweite Gegenstand Flaschenverschlüsse sind.

## Revendications

1. Procédé de stérilisation comprenant l'irradiation d'un premier objet à stériliser comprenant une partie évidée avec de l'oxygène réactif au niveau de la partie évidée, empiler un second objet à stériliser sur le dessus de celui-ci pour recouvrir la partie évidée de manière à former un espace fermé et à effectuer une stérilisation à l'intérieur de cet espace clos formé ;
**caractérisé en ce que** l'oxygène réactif est obtenu en générant du plasma.

2. Procédé de stérilisation selon la revendication 1, dans lequel l'oxygène réactif est obtenu en générant du plasma en utilisant un courant alternatif, et en générant de l'oxygène réactif à partir du plasma obtenu.

3. Procédé de stérilisation selon la revendication 1 ou 2, dans lequel l'oxygène réactif est généré par une réaction de plasma et de vapeur.

4. Procédé de stérilisation selon la revendication 3, dans lequel la vapeur est mélangée avec de l'air à un rapport de 0,2 à 2,5 de vapeur: air.

5. Procédé de stérilisation selon l'une quelconque des revendications 1 à 4, dans lequel le plasma est un jet de plasma.

6. Procédé de stérilisation selon l'une quelconque des revendications 3 à 5, dans lequel l'oxygène réactif a une température de 50°C à 80°C.

7. Procédé de stérilisation selon l'une quelconque des revendications 1 à 6, dans lequel la différence de température entre l'oxygène réactif et la surface du premier objet à stériliser est de 10°C ou plus.

8. Procédé de stérilisation selon l'une quelconque des revendications 1 à 7, dans lequel les premier et second objets sont des bouchons de bouteille.
